# EUROPEAN PATENT APPLICATION

(11) **EP 3 305 240 A1**
(43) Date of publication of application: **11.04.2018**
(21) Application number: 16799720.4
(22) Date of filing: 20.04.2016
(51) Int. Cl.: A61C 13/083, A61K 6/027

(54) **MATERIAL FOR DENTAL PROSTHESIS, BLOCK BODY FOR PRODUCING DENTAL PROSTHESIS, AND DENTAL PROSTHESIS**

(30) Priority: 25.05.2015 JP 2015105857
(71) Applicant: GC Corporation, Tokyo 113-0033 (JP)
(72) Inventor: HOSHINO, Tomohiro, Tokyo 174-8585 (JP); MASHIO, Go, Tokyo 174-8585 (JP); FUJIMOTO, Tatsuya, Tokyo 174-8585 (JP); YOSHINAGA, Masatoshi, Tokyo 174-8585 (JP); YOKOHARA, Hayato, Tokyo 174-8585 (JP); OHTA, Daisuke, Tokyo 174-8585 (JP); SATO, Takuya, Tokyo 174-8585 (JP)
(74) Representative: Müller-Boré & Partner Patentanwälte PartG mbB
(86) International application number: PCT/JP2016/062564
(87) International publication number: WO 2016/190012

(57) **Abstract**

In order to provide a material for forming a dental prosthesis which does not require further heat treatment after machining and can improve cutting ability even after obtaining necessary strength, the material for a dental prosthesis comprises 60.0 mass% or more and 80.0 mass% or less of SiO₂, 10.0 mass% or more and 20.0 mass% or less of Li₂O, and 5.1 mass % or more and 10.0 mass % or less of Al₂O₃.

## Description

### Technical Field

This invention relates to a material for dental prosthesis excellent in machinability, a block body for making dental prosthesis, and dental prosthesis.

### Background Art

With the recent development of CAD / CAM (Computer Aided Design / Computer Aided Manufacturing) technology, in making dental prosthesis, a shape of the dental prosthesis is handled with data converted into a predetermined format, transmitting the data to a processing apparatus, and the processing apparatus automatically selects machines such as cutting and grinding based on the data and produce the dental prosthesis. Thus, the dental prosthesis can be provided quickly.

For the dental prosthesis, it is necessary to have strength, hardness, chemical durability against the oral environment and aesthetic (color tone, texture) similar to natural teeth as basic functions for the dental prosthesis.

In addition to this, the dental prosthesis have complicated unevenness, and it is also important to machine a complicated shape in a short time without causing troubles such as chipping. The dental prosthesis can be produced more quickly by using a material that can be processed in such a short time.

Patent Literature 1 discloses a material for the dental prosthesis including a predetermined component, thereby improving the basic function and machinability.

### Citation List

### Patent Literature

Patent Literature 1: JP4777625B

### Summary of Invention

### Technical Problem

However, in an invention described in Patent Literature 1, machining is performed in a state where lithium metasilicate having excellent machinability is used as a main crystal phase, thereafter heat treatment is performed to obtain hard lithium disilicate. In this case, there is a possibility of deformation due to volumetric expansion and volumetric contraction accompanying further heat treatment after machining, and there is a problem that the final dimensional accuracy of dental prosthesis decreases.

In addition, when the heat treatment is performed, there is a problem that lithium disilicate becomes the main crystal phase and the dental prosthesis becomes hard, and then the machinability becomes poor. Even if this is processed, it is difficult to machine quickly.

In view of solving the above-mentioned problems, an object of the present invention is to provide a material for dental prosthesis which can obtain a necessary strength without applying further heat treatment after machining and has good machinability. Furthermore, a block body for the dental prosthesis using the above-mentioned material and a dental prosthesis are also provided.

### Solution to Problem

Hereinafter, the present disclosure will be described below.

One embodiment of the present disclosure is a material for forming a dental prosthesis comprising;
no less than 60.0 mass% and no more than 80.0 mass% of SiO₂,
no less than 10.0 mass% and no more than 20.0 mass% of Li₂O, and
no less than 5.1 mass % and no more than 10.0 mass% or less of Al₂O₃.

In the material for making the dental prosthesis, a main crystal phase may be lithium disilicate.

Here, the "main crystal phase" means a crystal phase having the largest crystal precipitation rate in the crystal phases observed by analysis with an X-ray diffractometer. The same is applied to the following.

The material for the dental prosthesis may further comprise at least one selected from the group consisting of no more than 2.8 mass% of Na₂O, no more than 10.0 mass% of K₂O, no more than 3.0 mass% of CaO, no more than 10.0 mass% of SrO, no more than 10.0 mass% of BaO, no more than 3.0 mass% of MgO, no more than 2.8 mass% of Rb₂O, no more than 2.8 mass% of Cs₂O, no more than 2.8 mass% of Fr₂O, no more than 3.0 mass% of BeO and no more than 10.0 mass% of RaO.

Also, it is a block body before making the dental prosthesis by machining and formed in a columnar shape whose material is formed of the above-mentioned material for the dental prosthesis.

Further, it is a dental prosthesis in a shape of the dental prosthesis and its material can provide the dental prosthesis comprising the above-mentioned material for the dental prosthesis.

### Advantageous Effects of Invention

According to the present invention, necessary strength can be obtained without applying a further heat treatment after machining and a material for a dental prosthesis having good machinability can be obtained. As a result, accurate dental prostheses can be provided promptly.

### Description of Embodiments

Hereinafter, the present invention will be described based on the embodiments. However, the present invention is not limited to these embodiments.

The block body for making a dental prosthesis according to one embodiment is in the form of a block having a columnar shape such as a prism, a cylinder or the like, from which it is deformed or scraped out by machining such as cutting or grinding to form a dental prosthesis. This block body for making the dental prosthesis is configured with a material for the dental prosthesis described later.

The dental prosthesis has a complicated shape and a part thereof is formed thin, and in order to machine-process such a shape quickly without causing chipping or the like with high accuracy, the material constituting the dental prosthesis have a great influence. On the other hand, the block body for making the dental prosthesis and the dental prosthesis according to this embodiment are formed of the following material for the dental prosthesis.

The material for the dental prosthesis according to this embodiment includes the following components. The main crystal phase of the material is lithium disilicate.
SiO₂: no less than 60.0 mass% and no more than 80.0 mass%
Li₂O: no less than 10.0 mass% and no more than 20.0 mass%
Al₂O₃: no less than 5.1 mass% and no more than 10.0% mass%

The above-mentioned respective components are as follows:
If the content of SiO₂ is less than 60.0 mass% or more than 80.0 mass%, it becomes difficult to obtain a homogeneous glass blank in the manufacturing process described later. It is preferably 65 mass% or more and 75 mass% or less.
If the content of Li₂O is less than 10.0 mass% or more than 20.0 mass%, it becomes difficult to obtain a homogeneous glass blank in the manufacturing process described later and machinability tends to decrease. It is more preferably 12 mass% to 18mass%.
If the content of Al₂O₃ is less than 5.1 mass%, lithium disilicate is precipitated as a main crystal phase but machinability tends to decrease. On the other hand, when the content of Al₂O₃ is more than 10.0 mass%, lithium disilicate is not the main crystal phase, and strength tends to decrease. It is more preferably 5.1 % mass% to 8.0 mass%.

Furthermore, the material for the dental prosthesis may contain the following components in addition to the above-mentioned components. However, as is apparent from the fact that contents of the component represented here includes 0 mass%, they do not have to be contained but any one of them may be contained.

A component for adjusting a melting temperature can be contained at 0 mass% or more and 15.0 mass% or less. This makes it possible to make the melting temperature appropriate in the manufacturing process described later. Although it may be contained more than 15.0 mass%, improvement of its effect is limited. Specific examples of melting temperature adjusting materials include oxides of Na, K, Ca, Sr, Mg, Rb, Cs, Fr, Be and Ra. More preferable materials are as follows:
Na₂O: no more than 2.8 mass%
K₂O: no more than 10.0 mass%
CaO: no more than 3.0 mass%
SrO: no more than 10.0 mass%
BaO: no more than 10.0 mass%
MgO: no more than 3.0 mass%
Rb₂O: no more than 2.8 mass%
Cs₂O: no more than 2.8 mass%
Fr₂O: no more than 2.8 mass%
BeO: no more than 3.0 mass%
RaO: no more than 10.0 mass%

In addition, the total amount of components for forming crystal nuclei can be 0 mass% or more and 10.0 mass% or less. As a result, a nucleus forming a lithium disilicate crystal is efficiently produced. However, since an improvement of the effect is limited even if more of the components are contained, the content is set to 10.0 mass% or less. As the compound functioning as a crystal nucleus forming material, oxides of Zr, P and Ti (ZrO₂, P₂O₅ and TiO₂) can be cited. In that case, at least one selected from ZrO₂, P₂O₅, and TiO₂ is contained, and the total content thereof is preferably 0 mass% or more and 10.0 mass% or less.

The material for the dental prosthesis may further contain a known colorant from the viewpoint of enhancing aesthetics. For example, V₂O₅, CeO₂, Er₂O₃ and the like can be mentioned.

Here, preferably, a void is not observed in a microphotograph at a magnification of 2000 times in a cross section of the material for the dental prosthesis. However, since some voids are considered to have small influence, preferably, area occupied by the voids in an observation range (for example, 60 µm in length × 60 µm in width) is 2% or less. Similarly, it is preferable that a granular material of the colorant is not observed in a microphotograph of the cut and polished surface of the dental prosthesis at a magnification of 200 times.

These voids and granular materials may form an interface with a base material, and which may affect machinability. Also, the presence of the granular material of colorant causes color unevenness of the dental prosthesis.

Such a material for the dental prosthesis can be certainly obtained by melt shaping as described later but not by powder shaping.

By the above-mentioned material for the dental prosthesis, the block body for producing the dental prosthesis and the dental prosthesis, basic functions as the dental prosthesis such as strength, hardness, chemical durability against the oral environment and aesthetic (color tone, texture) similar to natural teeth can be provided. In addition, machinability is also improved, and despite having sufficient strength that heat treatment after processing is unnecessary, the dental prosthesis can be machined without any defects under the same processing conditions as conventional ceramic materials for cutting.

Next, one example of a method for manufacturing the above-described dental prosthesis will be explained. A method for making the material for the dental prosthesis and a method for making the block body for the dental prosthesis are also included. The manufacturing method of this embodiment is configured to include a melting step, a glass blank manufacturing step, a nucleus forming step, a heat treatment step, a cooling step, and a processing step.

In the melting step, each components described above are melted at no less than 1300°C and no more than 1600°C. As a result, molten glass of the material for the dental prosthesis can be obtained. This melting is preferably carried out for several hours in order to obtain sufficiently uniform properties.

The glass blank making step is a step of obtaining the glass blank having a shape close to the shape of the block body for preparing the dental prosthesis. The molten glass obtained in the melting step is poured into a mold and cooled to room temperature to obtain the glass blank. In order to inhibit alteration or cracking of the material, the cooling is performed with a slow temperature change.

The glass blank thus obtained can also be supplied as a material for the dental prosthesis. In that case, the glass blank may be shaped in a form of a predetermined block to form the block body for making the dental prosthesis.

The nucleus forming step is a step of heating the glass blank obtained in the glass blank making step and maintaining the glass blank to be heated at no less than 400°C and no more than 600°C for a predetermined time. Thus, nuclei for crystal formation are formed. The maintenance time may be any time as long as the nucleus is sufficiently formed, so it is preferably no less than 10 minutes. The upper limit of the time is not particularly limited, but it can be set to no more than 6 hours.

The heat treatment step is a step of heating the glass blank without cooling and maintaining it at no less than 800°C and no more than 1000°C for a predetermined time. Thereby, a lithium disilicate blank in which the main crystal phase is lithium disilicate can be obtained. The upper limit of the time is not particularly limited, but it can be no more than 3 hours.

In the nucleus forming step and the heat treatment step, as described above, it is necessary to maintain the temperature within the predetermined temperature range, but it is not always necessary to maintain the temperature at a fixed temperature as long as it is within the predetermined temperature range. That is, the temperature may continue to be raised.

The lithium disilicate blank thus obtained can also be supplied as the material for the dental prosthesis. In that case, for example, the shape of the lithium disilicate blank may be arranged in the form of a predetermined block to form a block body for making the dental prosthesis.

In the heat treatment process, an intermediate process having different temperatures may be provided. That is, before maintaining at no less than 800°C and no more than 1000°C as described above, the glass blank is heated without cooling subsequent to the nucleus forming step, and is maintained, for example, at no less than 600°C and no more than 800°C for a predetermined time. Thus, the crystals can be produced and an intermediate can be obtained. The maintenance time in that case is preferably no less than 10 minutes. The upper limit of the time is not particularly limited, but it can be set to 6 hours or less. After this intermediate process, heating may be performed at no less than 800°C and no more than 1000°C as described above without cooling.

The cooling step is a step of cooling the lithium disilicate blank obtained by the heat treatment step to room temperature. This makes it possible to supply the lithium disilicate blank in the processing step.

The processing step is a step of machining the obtained lithium disilicate blank into a shape of the dental prosthesis. The method of machining is not particularly limited, but cutting and grinding can be mentioned. Thereby, the dental prosthesis can be obtained.

This processing can be performed under conditions with better productivity than before. That is, conventionally, the material for the dental prosthesis containing lithium disilicate as the main crystal phase has poor machinability, and therefore cannot be cut efficiently. Hence, conventionally, the materials were necessary to be processed without containing lithium disilicate as the main crystal phase in order to be processed easily, and necessary to go through a step of further strengthening afterwards by further heat treatment or the like.

On the other hand, according to the present invention, even when the material having lithium disilicate as the main crystal phase is used, cutting and grinding can be performed under conditions equivalent to those of conventional easy-machining materials. Since further heat treatment is not necessary after processing, the accuracy of machining can be maintained without changing the shape as the dental prosthesis.

### Examples

In Examples 1 to 9 and Comparative Examples 1 to 6, materials including lithium disilicate as the main crystal phase were prepared by changing the components in the above-described manufacturing method, and the dental prosthesis was produced by cutting, and machinability, strength, presence of voids and color unevenness at that time was evaluated. It is to be noted that Examples 1 to 9 and Comparative Examples 1 to 5 are produced by a melt molding method and Comparative Example 6 is produced by a powder molding method.

Table 1 and Table 2 show the contents of each component by mass%. Furthermore, Table 1 and Table 2 each show results of changing the components in the crystal phase (main crystal), machinability, strength, and the presence of voids and color unevenness. The blanks in the items of the components in Tables 1 and 2 represent 0 mass%.

The main crystal was measured by using X-ray diffractometer (Empyrean (registered trademark); manufactured by Spectris Co., Ltd.). As a result of quantitative analysis by the Rietveld method, among the observed crystal phase, the crystal phase having the highest crystal precipitation ratio was taken as the main crystal phase. In Table 1, "LS₂" represents lithium disilicate, and "LAS" represents lithium aluminosilicate.

As for machinability, two types of conventional materials for processing were prepared as Reference 1 and Reference 2. They are each the following materials:
(Reference 1) A material having lithium metasilicate as the main crystal phase ,and contains 72.3 mass% of SiO₂, 15.0 mass% of Li₂O and 1.6 mass% of Al₂O₃.
(Reference 2) A material having the crystal phase of lithium metasilicate and the crystal phase of lithium disilicate in approximately the same ratio, and the material contains 56.3 mass% of SiO₂, 14.7 mass% of Li₂O, and 2.1 mass% of Al₂O₃.

With regard to Examples and Comparative Examples, the processing time, consumption of the tool, and chipping with respect to the materials of Reference 1 and Reference 2 were evaluated respectively by processing with a ceramic processing machine (CEREC (registered trademark) MC XL; manufactured by Dentsply Sirona Inc.). In each case, those that were equivalent or better than the materials of Reference 1 and Reference 2 were shown as "good", and those which were not equivalent or less compared to the materials of Reference 1 and Reference 2 were shown as "bad".

The strength was evaluated by carrying out a biaxial bending test according to ISO 6872, and shown as "good" when the calculated biaxial bending strength was 300 MPa or more, and shown as "poor" when it was lower than 300 MPa.

The voids were evaluated by observing a cross section surface with a tabletop microscope (Hitachi High-Technologies Corporation, TM 3000) and analyzing the obtained image with image analysis software ImageJ. In the observation range of 60 µm in length × 60 µm in width, those having area occupied by voids of 2% or more were regarded as "Exist", and those having less than 2% were regarded as "None".

The color unevenness was evaluated by observing a cross section surface with a digital microscope (Keyence Corporation, VHX-2000) and checking the particles of the colorant within the observation range of 1 mm in length × 1 mm in width. At this time, the one in which particles of the colorant were observed was regarded as "Exist", and the one in which the particles of the colorant were not observed was regarded as "None".

**[Table 1]**

| | | Example 1 | Example 2 | Example 3 | Example 4 | Example 5 | Example6 | Example 7 | Example 8 | Example 9 |
|---|---|---|---|---|---|---|---|---|---|---|
| Component | SiO₂ | 63.0 | 65.2 | 73.3 | 71.2 | 75.3 | 64.7 | 70.6 | 76.2 | 75.7 |
| | Li₂O | 19.6 | 18.0 | 17.0 | 15.3 | 15.2 | 12.5 | 12.1 | 11.0 | 10.1 |
| | Al₂O₃ | 5.1 | 7.3 | 5.3 | 6.8 | 6.1 | 9.1 | 7.0 | 7.8 | 6.3 |
| | Na₂O | | 0.3 | 0.5 | | | 2.6 | | 1.7 | 2.3 |
| | K₂O | 2.7 | | 0.3 | 1.2 | 0.2 | 2.5 | 9.2 | | |
| | MgO | 2.1 | | 2.5 | | | | | | 0.3 |
| | CaO | | 2.8 | | | 1.2 | 0.4 | | | |
| | SrO | | 3.1 | 1.0 | | | 2.2 | | | |
| | BaO | 1.3 | | | | | 0.9 | | | 5.1 |
| | P₂O₅ | 4.1 | 1.5 | 0.1 | | 2.0 | 2.7 | 1.1 | | |
| | ZrO₂ | 2.1 | 0.1 | | 5.4 | | | | 3.1 | 0.2 |
| | TiO₂ | | 1.7 | | 0.1 | | 2.4 | | 0.2 | |
| Result | Main crystal | LS2 | LS2 | LS2 | LS2 | LS2 | LS2 | LS2 | LS2 | LS2 |
| | Machinability | Good | Good | Good | Good | Good | Good | Good | Good | Good |
| | Strength | Good | Good | Good | Good | Good | Good | Good | Good | Good |
| | Void | None | None | None | None | None | None | None | None | None |
| | Color unevenness | None | None | None | None | None | None | None | None | None |

**[Table 2]**

| | | Comparative Example 1 | Comparative Example 2 | Comparative Example 3 | Comparative Example 4 | Comparative Example 5 | Comparative Example 6 |
|---|---|---|---|---|---|---|---|
| Component | SiO₂ | 57.3 | 56.3 | 73.7 | 65.5 | 81.7 | 66.5 |
| | Li₂O | 15.4 | 15.5 | 13.8 | 14.5 | 10.6 | 11.6 |
| | Al₂O₃ | 12.8 | 1.5 | 3.5 | 11.3 | 2.5 | 13.3 |
| | Na₂O | | 1.0 | 5.6 | 3.2 | | 1.1 |
| | K₂O | 11.8 | 3.0 | | | 1.3 | 5.6 |
| | MgO | 0.2 | | | 0.4 | | |
| | CaO | | 0.5 | | | 3.4 | |
| | SrO | | 0.2 | | | | |
| | BaO | | | | 2.2 | | |
| | P₂O₅ | 1.0 | 6.0 | 3.4 | 1.3 | | 0.8 |
| | ZrO₂ | 1.5 | 14.0 | | 1.6 | | 1.0 |
| | TiO₂ | | 2.0 | | | 0.5 | 0.1 |
| Result | Main crystal | LAS | LS2 | LS2 | LAS | LS2 | LAS |
| | Machinability | Bad | Bad | Bad | Bad | Bad | Bad |
| | Strength | Good | Bad | Good | Bad | Bad | Bad |
| | Void | None | None | None | None | None | Exist |
| | Color unevenness | None | None | None | None | None | Exist |

As can be seen form Table 1, according to the material for the dental prosthesis of Examples, even when lithium disilicate (LS₂) is the main crystal phase, both machinability and strength are shown as good.

## Claims

1. A material for forming a dental prosthesis comprising:
No less than 60.0 mass% and no more than 80.0 mass% of SiO₂,
No less than 10.0 mass% and no more than 20.0 mass% of Li₂O, and
No less than 5.1 mass% and no more than 10.0 mass% of Al₂O₃.

2. The material for forming the dental prosthesis according to claim 1, wherein a main crystal phase is lithium disilicate.

3. The material for forming the dental prosthesis according to claim 1 or 2, further comprising at least one selected from the group consisting of no more than 2.8 mass% of Na₂O, no more than 10.0 mass% of K₂O, no more than 3.0 mass% of CaO, no more than 10.0 mass% of SrO, no more than 10.0 mass% of BaO, no more than 3.0 mass% of MgO, no more than 2.8 mass% of Rb₂O, no more than 2.8 mass% of Cs₂O, no more than 2.8 mass% of Fr₂O, no more than 3.0 mass% of BeO and no more than 10.0 mass% of RaO.

4. A block body for making a dental prosthesis, the block body before making a dental prosthesis by machining, wherein the block body is formed in a columnar shape, and the material of the block body for making the dental prosthesis is configured with any one of claims 1 to 3.

5. A dental prosthesis, wherein the dental prosthesis is in the shape of a dental prosthesis and whose material is any one of claims 1 to 3.
